# EUROPÄISCHE PATENTSCHRIFT

(11) **EP 1 007 502 B1**
(45) Veröffentlichungstag und Bekanntmachung des Hinweises auf die Patenterteilung: **07.11.2001**
(21) Anmeldenummer: 98948792.1
(22) Anmeldetag: 07.08.1998
(51) Int. Cl.: C07C 69/76, C10M 105/36, A61K 7/00, C10M 129/72

(54) **ESTER AROMATISCHER POLYCARBONSÄUREN MIT 2-ALKYLALKAN-1-OLEN**
ESTERS OF AROMATIC POLYCARBOXYLIC ACIDS WITH 2-ALKYLALKAN-1-OLS
ESTERS D'ACIDES POLYCARBOXYLIQUES AROMATIQUES AVEC DES 2-ALKYLALCAN-1-OLS

(30) Priorität: 29.08.1997 DE 19737793
(43) Veröffentlichungstag der Anmeldung: 14.06.2000
(73) Patentinhaber: SASOL Germany GmbH, 22297 Hamburg (DE)
(72) Erfinder: HERRMANN, Albert, Thomas, D-25541 Brunsbüttel (DE); FAHL, Jörg, D-21079 Hamburg (DE)
(74) Vertreter: Schupfner, Georg U.
(86) Internationale Anmeldenummer: DE9802343
(87) Internationale Veröffentlichungsnummer: WO9911599

(56) Entgegenhaltungen:
- EP-A- 0 157 583
- US-A- 4 543 420

## Beschreibung

Gegenstand der Erfindung sind Ester der Benzolpolycarbonsäuren mit verzweigten aliphatischen Alkoholen und deren Verwendung als Hydraulikflüssigkeit, als Schmiermittel oder als Zusatz in Kosmetika.

Ester sind als Schmiermittel seit langem bekannt. Sie werden in großem Stil z.B. in Flugmotoren als Schmieröle und als Schmierfette verwendet. Man verwendet vor allem die Umsetzungsprodukte einfacher Monocarbonsäuren mit C8- bis C10-Alkoholgemischen, wie sie bei der Oxosynthese, Hydrierung oder Aldol-Kondensation anfallen. Durch Veresterung mit Dicarbonsäuren und/oder Glykolen erhält man Komplexester, die den einfachen Esterölen zur Verbesserung der Viskosität, der Scherstabilität und des Schmierverhaltens zugesetzt werden. Esteröle finden auch in Schmierfetten wie Metallseifen oder Silikonen Verwendung.

Die typischerweise für die Veresterung eingesetzten verzweigten Alkohole sind z.B. durch die Oxosynthese erhältlich. Diese Alkohole sind chemisch nicht einheitlich und liegen als Isomerengemische vor.

Esteröle zeichnen sich durch niedrige Dampfdrücke, hohe Oxidationsstabilität, gutes Temperatur-Viskositätsverhalten und hohe Alterungsstabilität aus. Für bestimmte Anwendungen müssen sie eine gute Mischbarkeit mit dem gewünschten Betriebsmedium aufweisen. Betriebsmedium können etwa reine Kohlenwasserstoffe, Fluorchlorkohlenwasserstoffe oder Fluorkohlenwasserstoffe sein. Auch kann der Ester hinsichtlich des Tieftemperturverhaltens (Löslichkeit, Stockpunkt), sowie hinsichtlich der Hitzebeständigkeit (Flammpunkt) optimiert werden. Weitere Anforderungen ergeben sich durch die von der Anwendung her erforderliche Kompatibilität mit Kontaktmaterialien wie Kunststoffen, Metallen oder Farbüberzügen. Esteröle zeigen in der Regel auch ein gutes Hochdruck- und Lastaufnahmevermögen.

Esteröle aromatischer Polycarbonsäuren sind an sich bekannt. Die EP-A2- 0 157 583 beschreibt die Verwendung von Trimellitsäureestern, hergestellt aus linearen und/oder verzweigten primären Alkoholen, insbesondere C8- bis C10- Alkoholen. Die ausschließliche Verwendung verzweigter Alkohole führt nach der EP 0 157 583 zu einer unerwünscht hohen Viskosität und einer niedrigen Oxidationsstabilität.

Aufgabe der vorliegenden Erfindung ist es, Esteröle bereitzustellen, die besonderen Anforderungen hinsichtlich der Viskosität, des Viskositäts-Temperatur-Verhaltens und der Hochtemperaturbeständigkeit, insbesondere hinsichtlich der Oxidationsstabilität aber auch hinsichtlich der Hydrolysestabilität, gerecht werden und trotzdem ausgezeichnete Schmiermitteleigenschaften, insbesondere hinsichtlich der Lastaufnahme, haben.

Gegenstand der Erfindung sind Ester aromatischer Polycarbonsäuren mit 3 oder 4, Carboxylgruppen. Die erfindungsgemäßen Ester, bzw. deren Gemische lassen sich durch folgende Struktur beschreiben: 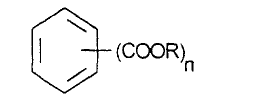 worin,
- n: eine ganze Zahl von 3 bis 4 ist und
- R: ein C12- bis C40- Kohlenwasserstoff-Rest folgender Struktur ist 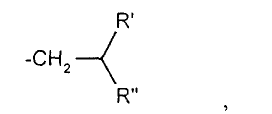
worin
- R': ein unverzweigter C6- bis C37- Kohlenwasserstoff-Rest ist und
- R": ein C1- bis C20- Kohlenwasserstoff-Rest ist,
wobei R, R' und R" für jedes n unterschiedlich sein können.

Vorzugsweise sind dies Ester der Trimellitsäure, Trimesinsäure und/oder Pyromellitsäure. Trimellitsäure wird auch als 1,2,4-Benzoltricarbonsäure bezeichnet und weist folgende Struktur auf: 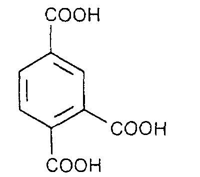 Trimesinsäure ist ebenfalls eine aromatische Tricarbonsäure und wird auch als 1,3,5-Tricarbonsäure bezeichnet: 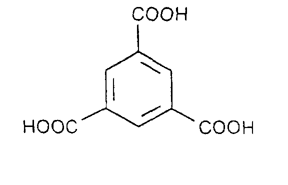 Pyromellitsäure wird auch als 1,2,4,5-Benzoltetracarbonsäure bezeichnet und hat folgende Struktur:

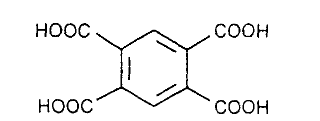

Die oben bezeichneten Säuren können durch Oxidation ggf. in Gegenwart von Katalysatoren wie Vanadiumpentoxid oder Mangan-Katalysatoren aus den entsprechend substituierten Polyalkylbenzolen gewonnen werden. Trimellitsäure- und Pyromellitsäureester sind besonders bevorzugt.

Die Alkoholgruppe weist eine 2-Alkylverzweigung auf. Die entsprechenden 2-Alkylalkan-1-ole sind z.B. durch die Guerbet-Reaktion oder die Oxosynthese zugänglich.

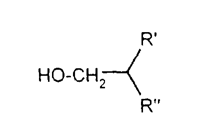

Vorzugsweise ist der Alkylrest (R) der Alkoholgruppe ein C12- bis C36-, besonders bevorzugt ein C12- bis C28-, Kohlenwasserstoff-Rest, wobei R' ein C6- bis C26-Kohlenwasserstoff-Rest, besonders bevorzugt ein C6- bis C22-Kohlenwasserstoff -Rest und R" ein C1- bis C18-Kohlenwasserstoff-Rest, besonders bevorzugt ein C3- bis C16-Kohlenwasserstoff-Rest ist. Beide Kohlenwasserstoff-Reste sind vorzugsweise gesättigte Reste. R" ist vorzugsweise ein unverzweigter (linearer) Kohlenwasserstoff.

Die genannten Ester sollen vorzugsweise unabhängig voneinander einen Flammpunkt (DIN ISO 2592) von über 270°C, einen Erstarrungspunkt von unter -40°C, vorzugsweise unter -45°C, und einen Viskositätsindex (DIN ISO 2909) von über 100 aufweisen.

Die erfindungsgemäßen Ester bzw. Zusammensetzungen enthaltend solche Ester finden Verwendung als Schmiermittel oder Schmiermittelzusatz, als Zusatz in Kosmetika oder als Hydraulikflüssigkeit zur Übertragung von Kräften. Namentlich genannt sei die Verwendung als Schmiermittel für Industriegetriebe, für die Metall- (z.B. als Walzöl), Kunststoff- oder Textilbe-/verarbeitung als Getriebeflüssigkeit und/oder Kühlschmierflüssigkeit (Kältemaschinenöl).

Die erfindungsgemäßen Ester sind besonders einfach aus den Anhydriden der aromatischen Polycarbonsäuren zugänglich, können aber auch direkt aus den Säuren hergestellt werden. Üblicherweise wird unter Zusatz eines Katalysators, wie einem Alkyltitanat, unter Wasserentzug verestert.

Die erfindungsgemäßen Ester können z.B in Kombination mit anderen Estern, inbesondere Neopentylpolyolen oder Silikonen als Schmiermittel eingesetzt werden. Ebenso können die erfindungsgemäßen Esteröle selbst als Betriebsmedium oder in Kombination mit anderen Betriebsmedien z.B. im Sinne einer Hydraulikflüssigkeit eingesetzt werden.

Soweit es sich trotz der guten Eigenschaften der erfindungsgemäßen Esteröle als zweckmäßig erweist, können Additive wie Verschleißverbesserer, VI-Verbesserer, Antioxidationsmittel, "High-Pressure"-Additive und Korrosionsinhibitoren, Dispergiermittel oder Metalldesaktivatoren zugesetzt werden.

### Beispiele

Für die Synthese wurden folgende 2-Alkylalkan-1-ole als Edukte eingesetzt:

**Tabelle 1**

| **2-Alkylalkan-1-ole** | | |
|---|---|---|
| Isofol® 12 | > 95 mol% | 2-Butyloctanol |
| Isofol® 14T | 10-20 mol% | 2-Butyloctanol |
| | 45-55 mol% | 2-Hexyloctanol / 2-Butyldecanol |
| | 25-35 mol% | 2-Hexyldecanol |
| | > 95 mol% | (Summe) |
| Isofol® 16 | > 97 mol% | 2-Hexyldecanol |
| Isofol® 20 | > 97 mol% | 2-Octyldodecanol |
| Isofol® 32 | > 90 mol% | 2-Tetradecyloctadecanol |

Isofol® ist eine eingetragene Marke der RWE-DEA Aktiengesellschaft für Mincraloel und Chemie. Die oben bezeichneten Alkohole sind kommerziell erhältlich als Produkte der Condea Chemie GmbH.

### 2-Alkylalkan-1-ol-Trimellitate

Trimellitsäureanhydrid wurde unter Zusatz von 0,15 Gew.% Isopropyltitanat als Katalysator mit den entsprechenden Alkoholen in 10 mol% Überschuß bei einer Temperatur von 170°C mit hohen Ausbeuten verestert. Nach Reindarstellung ergaben sich farblose, geruchlose Flüssigkeiten. Die Produkte lassen sich folgendermaßen charakterisieren:

**Tabelle 2**

| **Trimellitsäureester** | | | | | | |
|---|---|---|---|---|---|---|
| | | **T12** | **T14** | **T16** | **T20** | **T32** |
| **Kohlenstoffzahl** | | 45 | 51 | 57 | 69 | 105 |
| **Molgewicht** | [g/mol] | 714 | 810 | 882 | 1050 | 1566 |
| **Säurezahl** | [mg KOH/g] | 0,02 | 0,03 | 0,12 | 0,06 | 0,78 |
| **Verseifungszahl** | [mg KOH/g] | 236 | 209 | 193 | 150 | 107 |
| **Dichte bei 20°C** | [g/cm³] | 0,946 | 0,934 | 0,927 | 0,91 | 0,865 |
| **dynam. Viskosität** | bei 20°C [mPas] | 443 | 429 | 426 | 453 | 191 ⁺ |
| **kinemat. Viskosität** * | bei 20°C [mm²/s] | 395 | 403 | 403 | 437 | - |
| **kinemat. Viskosität** * | bei 40°C [mm²/s] | 122,6 | 129,6 | 133,3 | 148,3 | 226 |
| **kinemat. Viskosität** * | bei 100°C[mm²/s] | 13,5 | 14,9 | 16 | 18,4 | 26,1 |
| **Viskositätsindex** | | 106 | 117 | 127 | 139 | 148 |
| **Schmelzbereich** | [°C] | -46 | -45 | -48 | -48 | +19 |
| **Erstarrungsbereich** | [°C] | -47 | -46 | -48 | -48 | +16 |
| **Flammpunkt** | [°C] | 270 | 280 | 275 | 280 | 317 |
| **Smokepoint** | [°C] | 205 | 205 | 200 | 200 | 235 |
| **Hydrolysestabilität** | [%] hydrolysiert | 21,7 | 20 | 22,7 | 23,8 | 19,6 |
| **Säurezahl n. 7d 150°C** | [mg KOH/g] | 51,2 | 41,5 | 33,1 | 29 | 16,2 |
| *Die nachgestellte Zahl bezieht sich auf die Nummer des eingesetzten Isofols® und bezeichnet die C-Zahl der Alkoholgruppe. T steht für Trimellitat*. | | | | | | |

| | | | | | | |
|---|---|---|---|---|---|---|
| * *nach Ubbelohde,* ⁺ bei 40°C | | | | | | |

### 2-Alkylalkan-1-ol - Pyromellitate

Pyromellitsäureanhydrid wurde unter Zusatz von 0,15 Gew.% Isopropyltitanat als Katalysator mit den entsprechenden Alkoholen in 10 mol% Überschuß bei einer Temperatur von 170°C-180°C mit hohen Ausbeuten verestert. Die Produkte lassen sich folgendermaßen charakterisieren.

**Tabelle 3**

| **Pyromellitsäureester** | | | | | | |
|---|---|---|---|---|---|---|
| | | **P12** | **P14** | **P16** | **P20** | **P32** |
| **Kohlenstoffzahl** | | 58 | 66 | 74 | 90 | 138 |
| **Molgewicht** | [g/mol] | 926 | 1054 | 1150 | 1374 | 2036 |
| **Säurezahl** | [mg KOH/g] | 0,04 | 0,04 | 0,01 | 0,03 | 0,04 |
| **Verseifungszahl** | [mg KOH/g] | 243 | 221 | 197 | 165 | 103 |
| **Dichte bei 20°C** | [g/cm³] | 0.947 | 0,933 | 0,926 | 0,906 | 0,872 |
| **dynam.** Viskosität | bei 20°C [mPas] | 771 | 700 | 657 | 654 | 250 + |
| **kinemat. Viskosität** ***** | bei 20°C [mm²/s] | 709 | 658 | 625 | 650 | |
| **kinemat. Viskosität *** | bei 40°C [mm²/s] | 210 | 204 | 201 | 211 | 253 |
| **kinemat. Viskosität *** | bei 100°C[mm²/s] | 20,3 | 21,4 | 22,4 | 23,4 | 29,1 |
| **Viskositätsindex** | | 112 | 125 | 135 | 136 | 153 |
| **Schmelzbereich** | [°C] | -43 | -43 | -47 | -44 | 37 |
| **Erstarrungsbereich** | [°C] | -43 | -44 | -47 | -44 | 23 |
| **Flammpunkt** | [°C] | 265 | 275 | 273 | 292 | 315 |
| **Smokepoint** | [°C] | 195 | 235 | 235 | 157 | 170 |
| **Hydrolysestabilität** | [%] hydrolysiert | 24,1 | 23,8 | 22,7 | 23,8 | 19,6 |
| **Säurezahl n. 7d 150°C** | [mg KOH/g] | 43,8 | 38,1 | 33,1 | 29,0 | 16,2 |
| *Die nachgestellte Zahl bezieht sich auf die Nummer des eingesetzten Isofols*® *und bezeichnet die C-Zahl der Alkoholgruppe. P steht für Pyromellitat*. | | | | | | |

| | | | | | | |
|---|---|---|---|---|---|---|
| * *nach Ubbelohde,* ⁺ bei 40°C | | | | | | |

## Patentansprüche

1. Ester der Formel 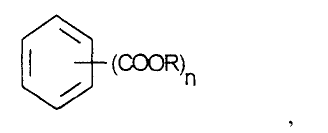 worin
n eine ganze Zahl von 3 bis 4 ist und
R ein C12- bis C40- Kohlenwasserstoff-Rest folgender Struktur ist 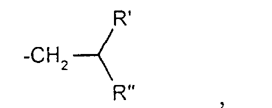
worin
R' ein unverzweigter C6- bis C37- Kohlenwasserstoff-Rest ist und
R" ein C1- bis C20- Kohlenwasserstoff-Rest ist,
wobei R, R' und R" für jedes n unterschiedlich sein können.

2. Ester gemäß Anspruch 1, worin die Säuregruppe Trimellitsäure, Trimesinsäure und/oder Pyromellitsäure ist.

3. Ester gemäß einem der vorhergehenden Ansprüche, worin
R ein C12- bis C36-Kohlenwasserstoff-Rest ist,
R' ein C6- bis C26-Kohlenwasserstoff-Rest und
R" ein C1- bis C18-Kohlenwasserstoff-Rest ist.

4. Ester gemäß einem der vorhergehenden Ansprüche, worin
R ein C12- bis C28-Kohlenwasserstoff-Rest ist,
R' ein C6- bis C22-Kohlenwasserstoff-Rest und
R" ein C3- bis C16-Kohlenwasserstoff-Rest ist.

5. Ester gemäß einem der vorhergehenden Ansprüche, worin
R' und R" gesättigte Kohlenwasserstoff-Reste sind.

6. Ester gemäß einem der vorhergehenden Ansprüche, worin
R" ein unverzweigter Kohlenwasserstoff-Rest ist.

7. Zusammensetzung enthaltend neben den Estern gemäß einem der Ansprüche 1 bis 6 keine anderen aromatischen Carbonsäureester.

8. Verwendung der Ester oder der Zusammensetzung gemäß einem der vorhergehenden Ansprüche als Schmiermittel oder Schmiermittelzusatz.

9. Verwendung des Ester oder der Zusammensetzung gemäß einem der Ansprüche 1 bis 7 als Hydraulikflüssigkeit.

10. Verwendung des Ester oder der Zusammensetzung gemäß einem der Ansprüche 1 bis 7 als Zusatz zu Kosmetika.

## Claims

1. An ester of the formula 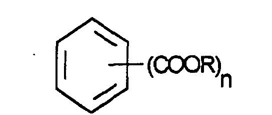 wherein
n denotes an integer from 3 to 4 and
R denotes a C12 through C40 hydrocarbon residue having the following structure: 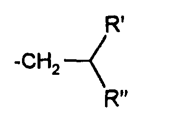
wherein
R' denotes an unbranched C6 through C37 hydrocarbon residue and
R" denotes a C1 through C20 hydrocarbon moiety,
where R, R' and R" may be different for each n.

2. An ester according to Claim 1, wherein the acid group is trimellitic acid, trimesic acid and/or pyromellitic acid.

3. An ester according to one of the preceding claims, wherein
R is a C12 through C36 hydrocarbon residue,
R' is a C6 through C26 hydrocarbon residue and
R" is a C1 through C18 hydrocarbon residue.

4. An ester according to one of the preceding claims, wherein
R is a C12 through C28 hydrocarbon residue,
R' is a C6 through C22 hydrocarbon residue and
R" is a C3 through C16 hydrocarbon residue.

5. An ester according to one of the preceding claims, wherein
R' and R" are saturated hydrocarbon residues.

6. An ester according to one of the preceding claims, wherein
R" is an unbranched hydrocarbon residue.

7. A composition which does not contain any other aromatic carboxylate ester in addition to the esters according to one of Claims 1 through 6.

8. A use of the esters or the composition according to one of the preceding claims as a lubricant or lubricant additive.

9. A use of the ester or the composition according to one of Claims 1 through 7 as a hydraulic fluid.

10. A use of the ester or the composition according to one of Claims 1 through 7 as a cosmetic additive.

## Revendications

1. Ester de formule 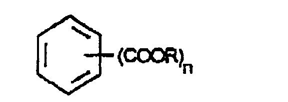 dans laquelle
n est un nombre entier de 3 à 4 et
R est un radical hydrocarboné en C₁₂ à C₄₀ de structure suivante 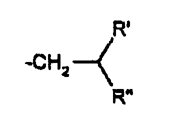
dans laquelle
R' est un radical hydrocarboné non ramifié en C₆ à C₃₇ et
R" est un radical hydrocarboné en C₁ à C₂₀,
R, R' et R" pouvant être différents pour chaque n.

2. Ester selon la revendication 1, dans lequel le groupement acide est l'acide trimellitique, l'acide trimésinique et/ou l'acide pyromellitique.

3. Ester selon l'une quelconque des revendications précédentes, dans lequel
R est un radical hydrocarboné en C₁₂ à C₃₆,
R' est un radical hydrocarboné en C₆ à C₂₆,
R" est un radical hydrocarboné en C₁ à C₁₈.

4. Ester selon l'une quelconque des revendications précédentes, dans lequel
R est un radical hydrocarboné en C₁₂ à C₂₈,
R' est un radical hydrocarboné en C₆ à C₂₂,
R" est un radical hydrocarboné en C₃ à C₁₆.

5. Ester selon l'une quelconque des revendications précédentes, dans lequel
R' et R" sont des radicaux hydrocarbonés saturés.

6. Ester selon l'une quelconque des revendications précédentes, dans lequel
R" est un radical hydrocarboné non ramifié.

7. Composition ne contenant, outre les esters selon l'une quelconque des revendications 1 à 6, pas d'autres esters d'acides carboxyliques aromatiques.

8. Utilisation des esters ou de la composition selon l'une quelconque des revendications précédentes comme lubrifiant ou additif de lubrifiant.

9. Utilisation de l'ester ou de la composition selon l'une quelconque des revendications 1 à 7 comme fluide hydraulique.

10. Utilisation de l'ester ou de la composition selon l'une quelconque des revendications 1 à 7 comme additif aux produits cosmétiques.
